# EUROPEAN PATENT APPLICATION

(11) **EP 4 789 786 A1**
(43) Date of publication of application: **12.08.2026**
(21) Application number: 26156369.6
(22) Date of filing: 04.02.2026
(51) Int. Cl.: B09B 3/35, B09B 3/40, B09B 3/45, F26B 3/36, A61L 2/00, A61L 11/00, B02C 18/00, B02C 18/12, E05D 3/02

(54) **A MACHINE FOR TREATING WASTE WITH A HEATED AIR SUPPLY ASSEMBLY**

(30) Priority: 07.02.2025 IT 202500002373
(71) Applicant: Ompeco S.r.l., 10051 Avigliana (TO) (IT)
(72) Inventor: LOMBARDO, Massimo, I-10051 Avigliana (Torino) (IT)
(74) Representative: Buzzi, Notaro & Antonielli d'Oulx S.p.A.

(57) **Abstract**

Disclosed herein is a machine (1) for treating waste including:
- a treatment cell (2) configured to receive waste for treatment in a treatment volume (V2) thereof, the treatment cell (2) comprising a main axis (Z2), a loading opening (4) which is closable and arranged at a top end (2T) thereof, and a discharge opening (5) which is closable and arranged at a bottom end (2B) thereof, the bottom end (2B) and the top end (2T) being opposite ends along said main axis (Z2),
- a rotor (7) arranged in the treatment volume (V2) of said treatment cell (2) at the bottom end (2B) thereof, the rotor (Z2) being operable in rotation about said main axis (Z2) to heat and shred the waste in said treatment volume (V2),
- a heated air supply assembly (8) having an inlet port (IN) and a delivery port (OUT), the delivery port (OUT) being in fluid communication with the treatment volume (V2), the heated air supply assembly (8) comprising an air feeder device (9) configured to draw in an air flow rate from an environment outside of the treatment volume (V2) and a heater device (10, R) configured to increase the temperature of said air flow rate prior to entering said treatment volume (V2).

## Description

### Field of the Invention

The present invention relates to the machines for treating waste, specifically to the machines for heating and stabilizing bioorganic as well as non-bioorganic waste. An example of such machines is described in the European Patent n. EP 1 546 623 B1.

### Prior Art

The machines for treating waste which the present invention refers to implement a thermal treatment, by means of locally generated heat and steam, of biological matter such as biomass, waste from food processing and, generally speaking, materials which contain or may contain biological substances, including living organisms. Moreover, it is possible to process non-organic and/or non-biological waste, such as e.g. plastics and metals. The treatment achieves a stabilization of the materials in the form of a sterilized and dehydrated product, which is stable for a long period of time.

Such machines generally comprise a treatment cell having a cylindrical or conical treatment volume (the latter type being described in the Italian Industrial Invention Patent Application n. 102020000030110), comprising a loading opening located at the top and a discharge opening in the vicinity of the bottom. The cell has a globally vertical axis, around which there is rotatably mounted a rotor operating within the treatment cell, typically near the bottom of said cell. The rotor advantageously comprises cutting profiles, which are configured to heat and shred the waste being treated within the treatment cell, wherein heating and shredding take place thanks to the combination of the friction between the rotor and the waste and of the dissipation of kinetic energy when the cutting profiles hit the waste. In some embodiments, such as the embodiment described in EP 1 546 623 B1, the heating action is enhanced and optimized by the presence of fixed profiles, which are adapted to cooperate with the cutting profiles on the rotor. The repeated interaction between the rotor and the waste leads to overheating the liquid fraction of the waste. After overheating, the machine is configured to dispense a controlled amount of water, in such a way as to drastically reduce the temperature and to achieve the devitalization of the bioorganic material which may be present in the waste.

Said machines are generally available on the market in a range of sizes which are determined by the capacity (volume) of the treatment cell. This implies that the machines of one and the same range essentially differ in size only, apart from a few dimensions which depend on structural strength requirements (for example the hub and the spokes of the rotor, or the cutting inserts on the rotor itself).

A technical problem which is common in this type of machines - not only in those described in the European Patent n. EP 1 546 623 B1 - regards the treatment of waste having a high moisture content, e.g. organic waste. In the treatment of such waste it is often possible to observe, after the initial shredding of the waste, a massive release of the moisture content within the treatment volume, with a consequent fluidization of the waste. This brings about two consequences:
i) Fluidized waste is more difficult to treat, since it tends to be shaken by the movement of the rotor, without however undergoing significant interactions useful for the treatment. In other words, fluidized waste becomes a mass spinning on itself and rotating relative to the treatment volume, dragged by the movement of the motor, which has the collateral effect of minimizing the impacts and the friction with the rotor, which increase the waste temperature;
ii) Waste with a high moisture content is regularly prone to thermally evolve towards temperatures lower than generally required for the thermal treatment and the stabilization of the waste, since the thermal energy developed in the interaction with the rotor is mainly converted into latent heat of evaporation of the huge amount of water contained in the waste. A treatment at lower temperatures may result, under given conditions, in waste which is not fully stabilized.

Both technical problems under consideration remain unsolved in the state of the art, wherein the proposed solutions generally consist in passively accepting said technical problems.

A further technical problem which the state of the art cannot but accept passively concerns the gradient of axial interference of the gasket arranged between the loading opening of the treatment cell of said machines and the lid which covers the loading opening (the gasket may be carried either by the lid or by the loading opening). The lid is generally hinged to the treatment cell or, more generally, to a fixed support and, as is commonly known, the relative position between the lid and the loading opening - where the gasket is located - is determined with high accuracy at the hinge, whereas it generally depends on the rigidity of the system in an amount which increases with the distance from the hinge. As a result, and also considering the counteraction offered by the gasket which works by compression in the axial direction with respect to the treatment cell when the lid is closed onto the loading opening, the surface of the lid is never perfectly parallel to the opposite surface of the loading opening, with which it implements the axial compression of the gasket; on the contrary, the lid tends to have a distance which varies from a minimum value at the hinge to a maximum value in the position farthest from the hinge. As a consequence, the interference with the gasket is highest in the vicinity of the hinge and lowest in the remotest position, often leading to an excessive interference in the vicinity of the hinge and to an early wear of the gasket.

### Object of the Invention

The invention aims at providing a machine for treating waste which is adapted to treat waste with a high moisture content without incurring in the effects of waste fluidization, and generally in the effects due to the high content of moisture, which have been described in the foregoing (at i) and ii). Secondarily, a further object of the invention consists in uniformizing the interference between the lid and the gasket at the loading opening of the treatment cell of the machine for treating waste.

### Summary of the Invention

The object of the invention is achieved by means of a machine for treating waste having the features set forth in the claims that follow, which form an integral part of the technical disclosure provided herein in relation to the invention.

### Brief Description of the Figures

The invention will now be described with reference to the annexed Figures, which are provided by way of non-limiting example only and wherein:
- Figure 1 is an overview of a machine for treating waste according to the invention,
- Figure 2 is a schematic of the circuits of a part of the machine, which illustrates the invention,
- Figure 3 is an isolated perspective view of a portion of the treatment cell of the machine according to the invention,
- Figure 4 corresponds to the view of Figure 3, but with some components removed in order to show internal parts of the treatment cell,
- Figure 5 is a partially exploded view corresponding to Figure 4,
- Figure 6 is an isolated view of some components of the treatment cell of the machine according to the invention,
- Figure 7 is a sectional view along a diametral plane of the treatment cell,
- Figure 8 shows a further aspect of the machine according to the invention, and
- Figure 9 shows a detail along arrow IX in Figure 8.

### Detailed Description

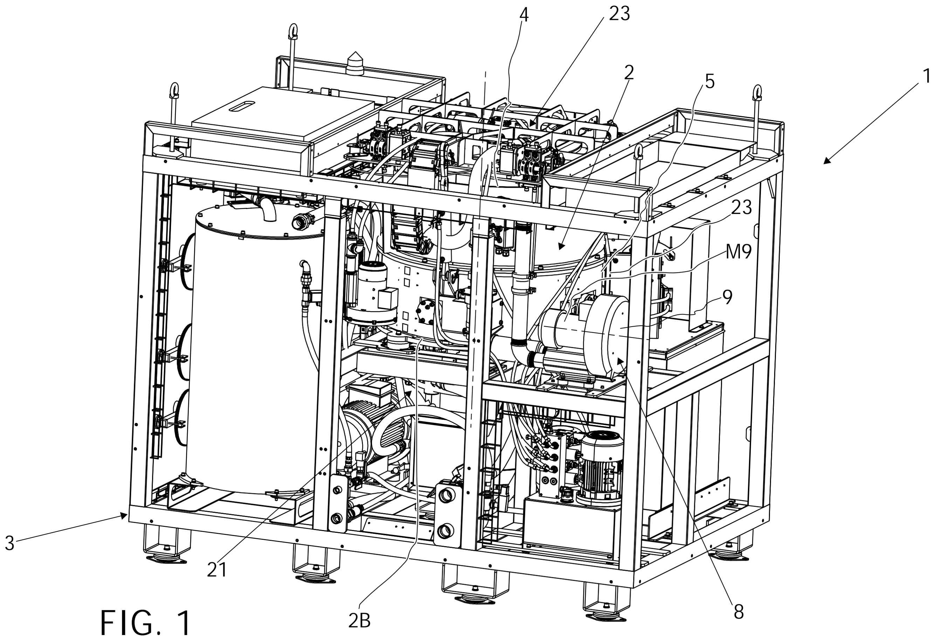

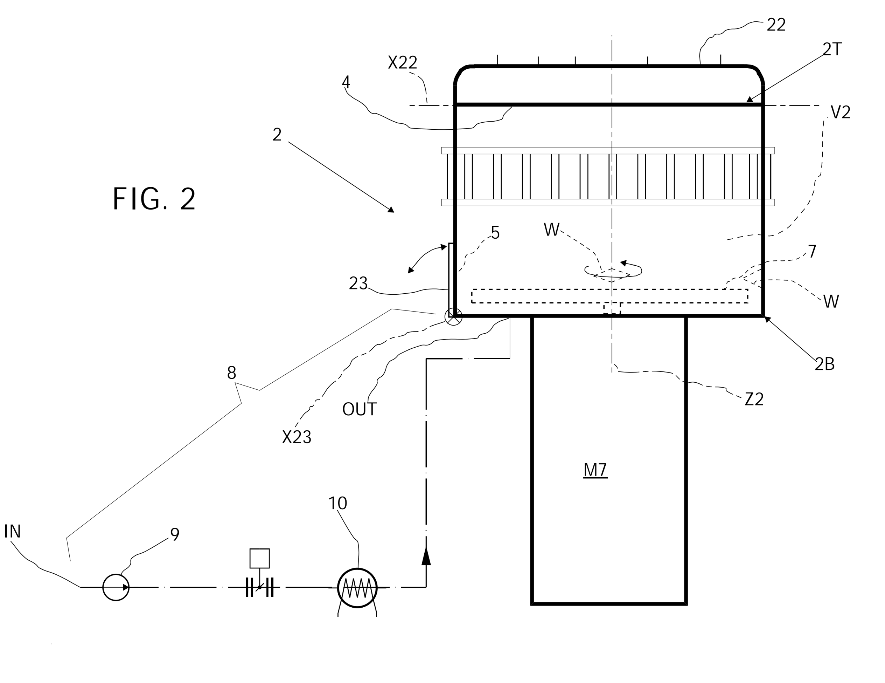

Reference 1 in Figure 1 generally denotes a machine for treating waste according to the invention. Referring to Figures 1 and 2, in various preferred embodiments, the machine 1 comprises a treatment cell 2 which is configured to receive waste for treatment (specifically thermal treatment and stabilization) in a treatment volume V2, and which has a main axis Z2 arranged vertically. The treatment cell 2 is installed on a frame 3 carrying all further components of the machine 1, and it comprises a loading opening 4 which is closable and arranged at a top end 2T, and a discharge opening 5, which in turn is closable and arranged at a bottom end 2B, wherein the bottom end 2B and the top end 2T are opposite ends 2 along the main axis Z2. The discharge opening 5 is preferably provided on a side wall 6 of the cell 2, which wall delimits the volume V2 and extends from the end 2B to the end 2T. The treatment volume V2 has a generally cylindrical shape and comprises an inner diameter extending in a direction transverse relative to the main axis Z2 and being generally constant along said main axis Z2 towards the bottom end 2B. In some embodiments, however, the volume V2 may have a conical (frustoconical) shape, the inner diameter thereof decreasing along said main axis Z2 towards the bottom end 2B, specifically decreasing from a maximum value at the top end 2T to a minimum value at the bottom end 2B.

In various embodiments, including the embodiment shown in the Figures, the inner diameter of the treatment volume V2 decreases at a constant rate. This means that the treatment volume V2 (and, in the present case, also the cell 2 as a whole) comprises a conical wall 6 the diameter thereof decreases in a manner directly proportional to the position along the axis 22. Specifically, preferred values of the coefficient of diameter reduction as a function of the axial position are comprised between 0.2 and 0.3.

In other embodiments, the inner diameter of the treatment volume V2 decreases at a non-constant rate. In other words, the treatment volume V2 comprises a wall the diameter whereof decreases in a fashion that does not meet a relation of direct proportionality between the axial position of the inner diameter and the value of the same diameter.

In still further embodiments, the inner diameter may decrease towards the bottom end 2B for a part of the extension of the treatment volume V2 along the axis Z2. The decrease may be at a constant or at an inconstant rate, and the portion(s) which are not involved in the decrease may have a constant inner diameter or else an increasing inner diameter, from the top end 2T towards the bottom end 2B, provided that said portions (or said portion) be necessarily positioned above - or in any case, relative to the end 2T, in closer vicinity to - the portion wherein the diameter decreases towards the bottom end 2B.

The machine 1 further comprises a rotor 7 arranged in the treatment volume V2 of the treatment cell 2 at the bottom end 2B thereof. The rotor 7 is operable in rotation about the main axis Z2, in order to heat and shred waste in the treatment volume V2 during the use of the machine 1. Specifically, the rotor 7 comprises cutting elements configured to heat and shred the waste in the treatment volume V2 during the use of the machine 1. The rotor 7 is schematically represented in all the Figures (sometimes in uniformly dotted lines and sometimes in dash-dotted lines) since it is known per se and since its geometry varies as a function of the applications. In this regard, the rotor 7 is driven in rotation by means of an electric motor (or, in some applications, a gearmotor) M7. The motor (or gearmotor) M7 is carried by the frame 3. In a way known per se, the rotor 7 comprises at least two spokes (typically two, but some applications may envisage more than two spokes) whereon cutting inserts and/or shredders are arranged. In an embodiment, the rotor comprises a pair of wedge-shaped cutting inserts at the leading edge of each of the spokes, wherein the "leading edge" is the edge which first contacts the waste during the rotation of the rotor 7. In other embodiments, it is possible to envisage the presence of inserts on the leading edge and of the trailing edge, in such a way to enable the rotor to shred and heat the material in both rotation directions (clockwise and anticlockwise). Moreover, in some embodiments the cutting inserts on the rotor are configured to cooperate with fixed wedge-shaped profiles W or generally with fixed blades arranged in a circular crown on the wall 6 of the treatment volume V2, and/or on the bottom of the cell itself. An example of such structure is described in EP 1 546 623 B1.

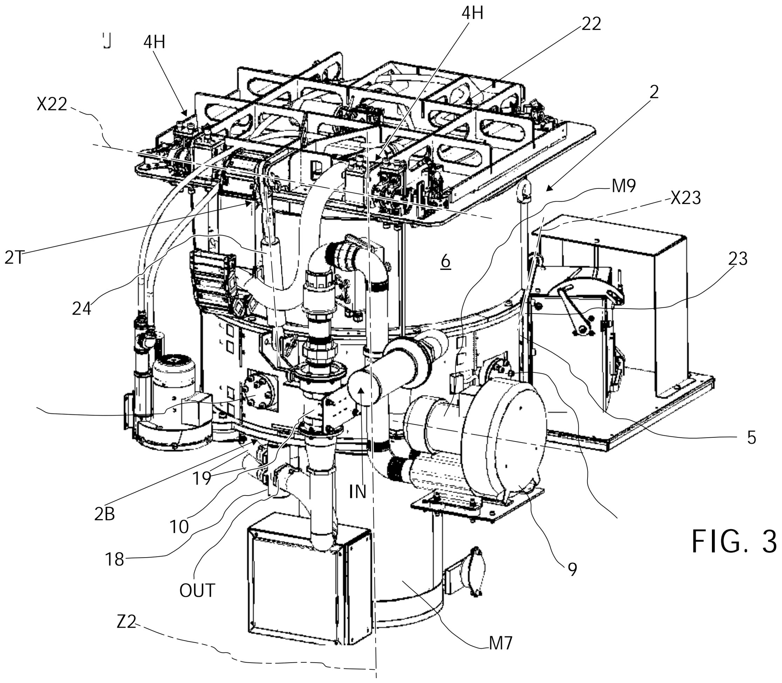

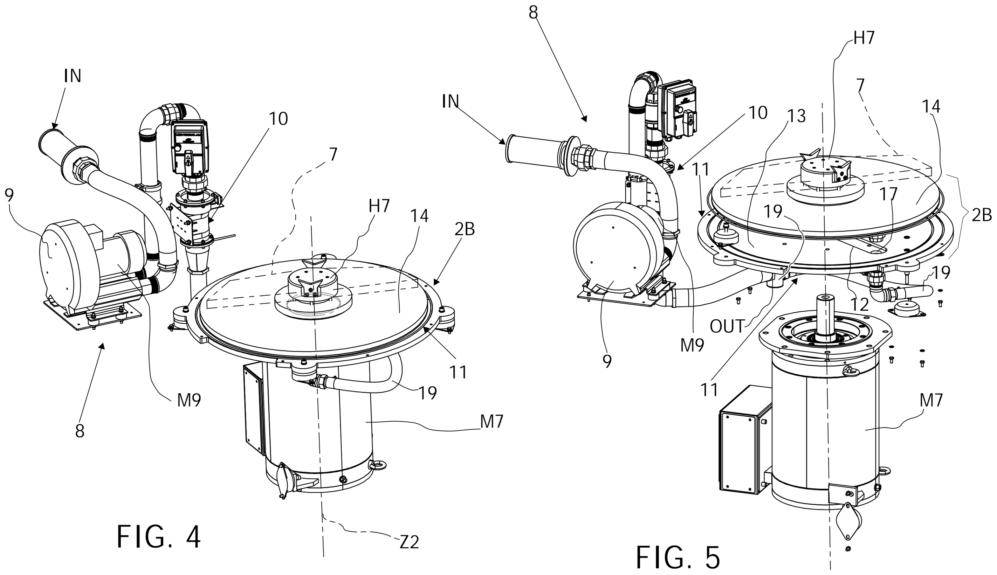

According to the invention, the machine 1 comprises a heated air supply assembly 8 having an inlet port IN and a delivery port OUT, wherein the delivery port OUT is in fluid communication with the treatment volume V2. The heated air supply assembly 8 comprises an air feeder device 9, preferably a fan actuated by an electric motor M9 (Figures 3-5), which is configured to draw in an air flow rate from an environment outside of the treatment volume V2, and a heater device 10 (preferably an electric heater) configured to increase the temperature of the air flow rate processed by the device 9 prior to entering the treatment volume V2. Preferably, the heater device 10 is an element separated from than the air feeder device 9, and it is arranged downstream thereof. However, in some embodiments it is possible to integrate the air feeder device 9 and the heater device 10 into a single component.

In preferred embodiments of the invention, referring to Figure 2 (and, generally speaking, to the Figures annexed herein), the delivery port OUT is in fluid communication with the treatment volume V2 at the bottom end 2B. In the preferred embodiment shown in Figures 1 and 3 to 6, the delivery port OUT is in fluid communication with the treatment volume V2 at a hub H7 of the rotor 7.

In the preferred embodiment under consideration, which will presently be described with reference to Figures 3 to 7, the introduction of the heated air flow rate into the volume V2 is implemented by making use of a layered construction of the bottom end 2, which has the further technical advantage (beside the main advantage of introducing the heated air flow rate into the volume V2, which is the base of the invention) of enabling the retrofitting of existing machines, so that the present invention may be implemented thereon.

In greater detail, the bottom end 28 comprises:
- a lower disc 11 including at least one channel 12 extending towards the main axis Z2 and formed within a thickness T11 (extending along the direction of the axis 22) of the lower disc 11, wherein each channel 12 is in fluid communication with the delivery port OUT of the heated air supply assembly 8, is open at an upper face 13 of the lower disc 11 and leads out at a first through-hole B11 of the lower disc 11 coaxial to the main axis Z2,
- an upper disc 14 arranged above the lower disc 11 in such a way that a lower face 15 of the upper disc 14 faces the upper face 13 of the lower disc 11, superiorly delimiting each channel 12, wherein the upper disc encompasses a second through-hole B14 coaxial to the main axis Z2,
- a sleeve element 16 arranged coaxially to the main axis Z2, wherein the sleeve element 16 is housed in a sequence of holes comprising the first through-hole B11 of the lower disc 11 and the second through-hole B14 of the upper disc, and further comprising one or more distributor holes 16 being in fluid communication with each channel 12 of the lower disc 11, so that each distributor hole 16 is configured for the introduction of the air flow rate supplied by the heated air supply assembly 8 into the treatment volume V2 through the delivery port OUT; the sleeve element 16 is traversed by an output shaft S7 of the electric motor M7 of the machine 1, and the shaft S7 is connected in rotation to the hub H7 of the rotor 7 for rotational drive of the rotor 7 itself.

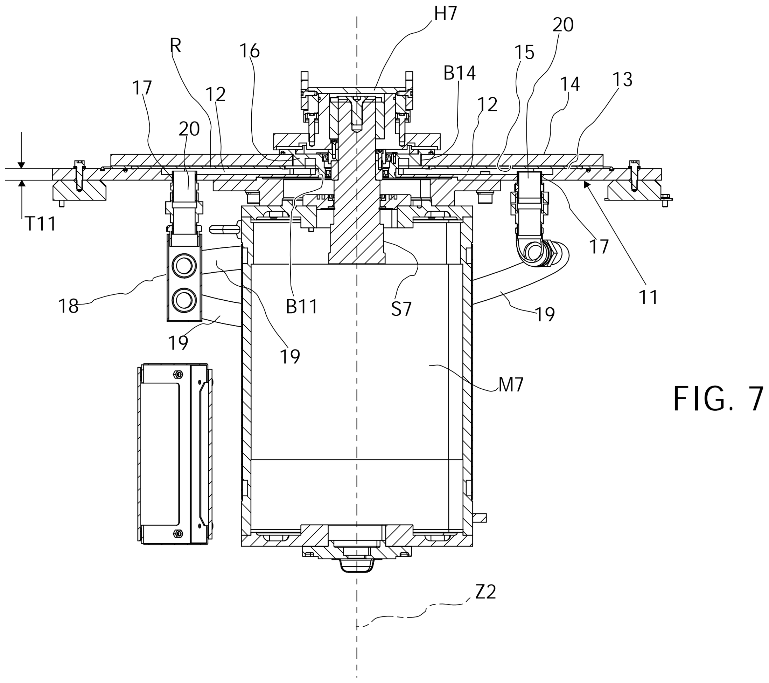

Preferably, a sheet-like resistor having a disc shape, identified by the reference R (see the section in Figure 7) is arranged between the disc 11 and the disc 14, superiorly delimiting each channel 12 and using the disc 14 as a shoulder to limit the deformations of the resistor R when the heated air enters the channels 12. The resistor R, if present, acts as a further or as the only heater for the flow rate processed by the air feeder device 9, thus operating in synergy with the heater device 10 when both are present, and taking the local heating action to the channels 12. Generally speaking, therefore, in the embodiments of the invention the heater device may include at least one of:
- the heater 10, preferably an electric heater, which is arranged downstream of the air feeder device 9 along the fluid communication with the treatment volume V2,
- the sheet-like resistor R arranged between the lower disc 11 and the upper disc 14.

Always referring to the heated air supply assembly 8, the heater 10 (preferably an electric heater) mainly operates by heating the air which is introduced into the volume V2 thereby decreasing the relative moisture content thereof, thus sending to the inlet of the volume V2 an air flow rate which is adapted to absorb the moisture (water) released by the waste subjected to treatment in the volume V2.

The sheet-like resistor R operates in a similar fashion (if needed, by providing further heating in addition to the heating by the heater 10, if both are present), and has the further effect of heating by conduction the bottom end of the cell 2, i.e. the assembly of the discs 11 and 14 which the sheet-like resistor R contacts. This enables, i.a., an immediate increase in efficiency of the machine 1 at cold start, thus making the machine 1 efficient already during the first treatment cycle, even in the presence of waste with a high moisture content.

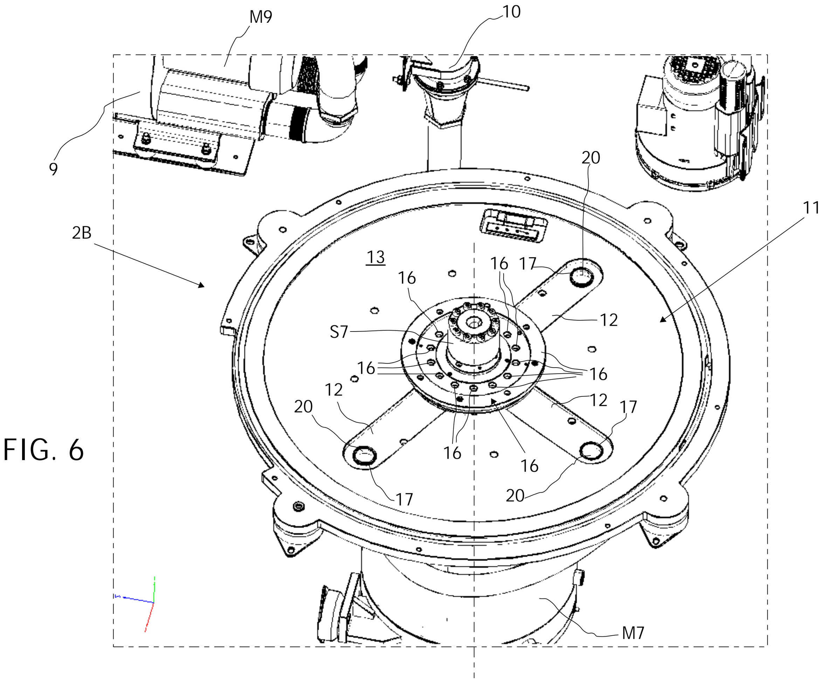

Always referring to the Figures 3 to 7 (and specifically to the Figures 6, 7), in the preferred embodiment the lower disc 11 comprises three channels 12 extending along radial directions and defining a substantial T shape, with two channels 12 being aligned along a diametral trajectory and arranged on opposite sides with respect to the hole B11, and a third channel 12 orthogonal to both. The fluid communication between the delivery port OUT and the channels 12 is implemented by the presence of admission lumens 17 at the end of each channel 12 which is radially farther from the axis Z2. The distribution of the air flow rate which has been heated by the delivery port OUT to the lumens 17 is preferably implemented by connecting the delivery port OUT to an inlet of a manifold 18 having as many outlets as the number of the lumens 17 on the disc 11. The fluid connection preferably takes place by means of duct sections 19, each of which branches out from a respective outlet of the manifold 18 and ends at a corresponding lumen 17 with a fluidic connection 20, e.g. a quick coupling connection. In this fashion, the heated air flow rate entering from the lumens 17 traverses the channels 12 in a centripetal direction, and enters the treatment volume spreading through the holes 16 which are arranged around the hub H7 of the rotor 7 (or which anyway follow the contour from the outside or from the inside thereof). The rotor is located above the arrangement of holes 16, and therefore during the rotation the heated air flow rate is introduced below the rotor, in correspondence with the hub, thus permeating the waste mass being treated from below.

In other embodiments, it is in any case possible to have the delivery port OUT in fluid communication with the treatment volume V2 at the side wall 6 of the treatment cell 2, which wall is located between the bottom end 2B and the top end 2T.

Whatever the embodiment, and in a way known per se, the machine 1 further comprises:
- a negative pressure fluid circuit being in fluid communication with the internal volume V2 of the treatment cell 2, and being controllable by means of a supply assembly configured to establish a negative pressure within the treatment volume V2, wherein the supply assembly preferably comprises a vacuum pump 21 (for example a liquid ring vacuum pump), and
- a circuit for devitalization water metering and for temperature control, which is preferably supplied with water from the mains, being in fluid communication with the volume V2 of the cell 2.

In greater detail, the circuit for devitalization water metering and for temperature control is in fluid communication with the volume V2 by means of a nozzle located in the vicinity of the loading opening 4, in such a way as to hit the waste from above downwards.

In order to perform the waste treatment in the volume 2 at sub-atmospheric pressure, the loading opening 4 and the discharge opening 5 are hermetically closable by means of a lid 22 and a hatch 23, respectively (see Figures 1-3). Both the lid 22 and the hatch 23 may be moved from a closing position, wherein they abut the openings 4 and 5 (respectively), thus ensuring fluid tightness thereat, and an opening position, wherein they are raised or anyway removed from the respective opening, so as to enable the transit of material.

In this regard, the negative pressure fluidic circuit is in fluid communication with the volume V2 of the treatment cell 2 by means of the lid 22, which carries a suction port (which is preferably shielded by a grid, so as to prevent big-sized debris from entering the negative pressure circuit) being in fluid communication with the volume V2, so as to enable extracting the air contained therein.

The lid 22 and the hatch 23 are operated by means of respective electromechanical actuators about axes transverse to the axis Z2, which are identified by the references X22 (for the lid 22) and X23 (for the hatch 23). The actuator which operates the lid 22 is identified with the reference number 24 in Figure 3, whereas the actuator which operates the hatch 23 is not visible in the Figure, but it is known per se. As will be described below, the axis X22 is practically a floating hinge axis for the lid 22, in a preferred embodiment of the invention.

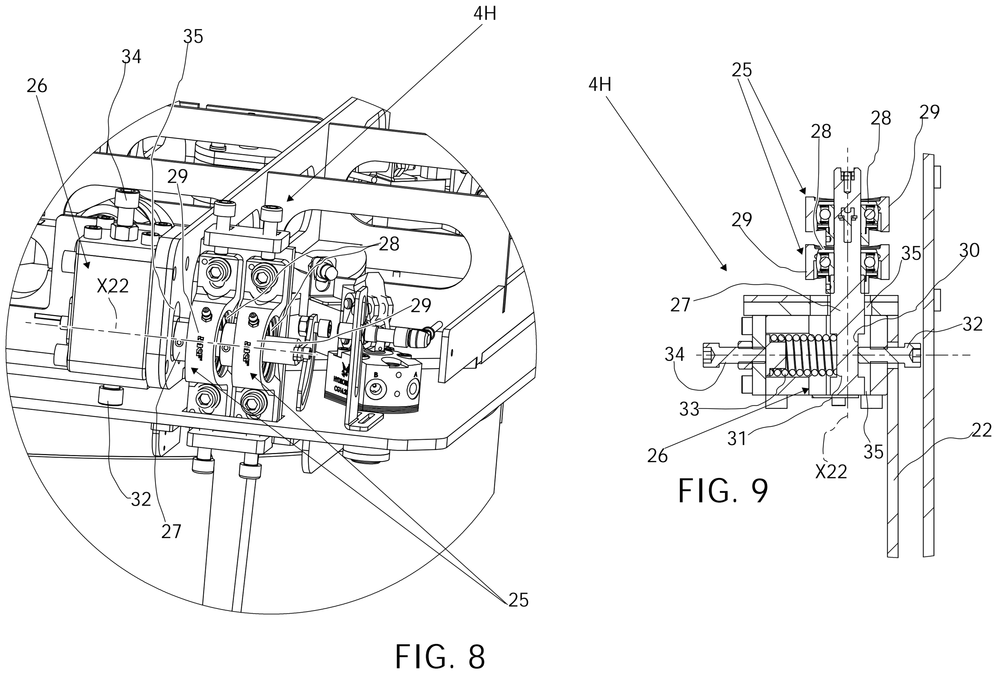

Referring to Figures 8 to 9, according to an advantageous aspect of the invention, the lid 22 is hinged to a fixed support, particularly to the treatment cell 2 (but it might as well be an element of the frame 3) by means of at least one lid hinge 4H. Each lid hinge 4H includes:
- a first hinge element 25 provided on the fixed support,
- a second hinge element 26 provided on the lid 22,
- a hinge pin 27 received in the first hinge element 25 and in the second hinge element 26, pivotally connecting them,
wherein the hinge pin 27 is received in a floating manner in a direction transverse to a longitudinal axis thereof, which coincides with the hinge axis X22, in at least one of the hinge elements 25, 26.

In the preferred embodiment shown in the Figures, the first hinge element 25 comprises one or more rolling bearings 28 - in the present case in the number of two, which are arranged side by side - connected to the fixed support, specifically by means of straddle-like supports 29, wherein the rolling bearings 28 rotatably support the hinge pin 27 and are fixed in a transverse direction relative to the longitudinal axis X22 of the hinge pin 27. In other words, in the preferred embodiment the floating arrangement of the hinge pin 27 is implemented only with respect to the second hinge element 26, whereas the coupling between the hinge pin 27 and the first hinge element 25 is rigid (obviously, apart from the rotation of the hinge pin 27 about the axis X22).

As regards the second hinge element 26 (Figure 9), the floating arrangement of the hinge pin 27 is preferably implemented by blocking the rotation of the pin 27 about the axis X22 relative to the hinge element 26. As can be seen in Figure 7, the hinge pin 27 comprises a pair of diametrically opposed flattenings 30, 31, wherein on the first flattening 30 a first screw 32 abuts which blocks the rotation of the pin 27 relative to the hinge element 26, and wherein on the flattening 31 a spring element 33 abuts which counteracts the movement of the hinge pin in the direction transverse to the axis X22, and the preload whereof is adjustable by means of a second screw 34. As clearly visible in Figure 9, moreover, the first screw 32 acts as stroke limit for the movement of the hinge pin in a direction transverse to the axis X22.

In order to guide the movement of the hinge pin 27 relative to the hinge element 26, the second hinge element 26 comprises a guide profile 35 extending in a direction transverse to the longitudinal axis X22 of the hinge pin, preferably comprising a pair of slots 35 traversed by the hinge pin 27 and arranged on opposite sides relative to the spring element 33. In this regard, the second hinge element 26 comprises a prismatic casing including the guide profile, thus preferably including two slots 35 on two opposite walls thereof and receiving the pin 27. Alternatively, it is possible to receive the pin 27 within a slider, which in turn is arranged within the element 26 and is guided by the guide profile 35. In this case, the slider may in turn include one or more rolling bearings, so that - albeit being fixed in rotation relative to the casing of the hinge element 26 - the pin 27 is allowed to rotate (also) relative to the hinge element 26.

Each hinge 4H enables closing the loading opening 4 by means of the lid 22 while equalizing the condition of interference between the lid 22 and one or more gaskets which provide fluid tightness between the lid and the loading opening 4 by means of a compression exerted by the lid 22. The floating arrangement of the pin 27 enables reducing the condition of interference between the lid 22 and the gasket at each hinge 4H, by aligning it to the one which acts along the remaining portions of the circumference where the lid and the gasket contact each other. In other words, the floating arrangement of the pin 27 within the hinge element 26 enables reducing the amount of interference between the lid 22 and the gasket, by converting the excessive interference into a compression of the spring element 33, with a consequent slight rising of the lid 22 at each hinge 4H when the lid is being completely closed, in comparison with the position which would geometrically result in the case of a hinge without floating pin. This ensures achieving a more uniform condition of parallelism between the lid 22 and the loading opening 4, and therefore a more uniform interference between the lid 22 and the gasket at the loading opening. As a result, the fluid tightness is better in comparison with the known solutions, and the useful life of the gasket is sensibly prolonged, since it is possible to eliminate one of the main reasons for failure, which is connected to excessive interference at each hinge. Finally, it must be observed that the gasket(s) 4H may be applied to any machine for treating waste of the same type of the machine 1, irrespective of the presence therein of the heated air supply assembly 8, and it is also applicable to any machine for treatment (even without rotor) which comprises a treatment cell closable by a lid (specifically hermetically closable, thanks to the interposition of a gasket), since the advantages as regards the uniformity of the interference between the treatment cell and the lid may be extended directly to such machines.

The operation of the machine 1 is as follows.

The machine functionally operates as a machine for treating (shredding, heating and stabilizing) waste of the known type. The waste to be processed, whatever its nature, is poured into the volume V2 of the treatment cell 2 through the loading opening 4, which is made accessible to this purpose by raising the lid 13 by means of the respective actuator. The discharge opening 5 is kept closed by means of the hatch 23.

Once the loading of the waste into the volume V2 is completed, the opening 4 is closed by applying and hermetically sealing the lid 22, and the work cycle is started. This envisages, first of all, the establishment of a negative pressure within the volume V2, by activating the vacuum pump 21 which, through the fluidic connection to the lid 22, draws out the air contained in the volume V2 through the suction port which is located on the same lid 22.

Subsequently, the rotation of the rotor 7 is started: the interaction between the waste and the rotor 7 rotating about the axis Z2 (if necessary, with the further crushing of the waste implemented by the organs W) leads to shredding and overheating the waste within the volume V2, up to temperatures which, in the case of dry waste, may reach 150°C or even 180°C. During this step the heated air flow rate, processed by the supply assembly 8 and sent to the treatment volume 2 through the delivery port OUT and - in the preferred embodiment - the admission lumens 17, supports the evaporation of the moisture that the waste has released in the meantime, in particular if the latter is organic waste with a high moisture content. By adjusting the thermal power delivered by the heater device 10 it is possible to regulate the temperature of the heated air flow rate entering the volume V2, adapting it to the evaporation needs which derive from the type of the waste being treated. This enables, i.a., avoiding both consequences i) and ii) discussed at the beginning of the description, and therefore it enables preventing the mass of treated waste from spinning on itself and preventing a treatment at excessively low temperatures. Moreover, the delivery of a heated air flow rate to the volume V2 during the treatment enables avoiding an operation at excessively low negative pressures, thereby reducing the stresses on the machine 1. In the absence of a heated air flow rate, as is the case in the known solutions, it is often necessary to operate at very negative pressures in order to foster evaporation, with consequent heavy mechanical stresses on the treatment cell 2. In this regard, the Applicant has observed significant beneficial effects in the treatment of waste with a high moisture content already with moisture contents of 30% of the total weight of the waste, and has ascertained the presence of such benefits also in the treatment of waste with a moisture percentage of 70% of the total weight of the waste.

At the end of the overheating step, the shredded waste mass is stabilized by adding a controlled amount of water through the nozzle which projects out of the lid 22. Preferably, the water flow rate is adjusted by means of a solenoid valve upstream of the nozzle (or incorporated in the same nozzle) which, as described in the foregoing, is preferably supplied with water from the mains. In this manner, the waste which has previously been sterilized and is substantially devoid of water components is stabilized, bringing about the devitalization of the bioorganic components possibly remaining therein.

The waste treatment is thus terminated: the rotor 7 is stopped and the hatch 23 is opened, thus exposing the discharge opening 5. The rotor 7 is briefly reactivated in order to expel the treated waste through the opening 5, collecting it in a tray housed in the frame 3 of the machine or in a bag, which may then be vacuum sealed.

Thanks to the heated air supply assembly 8 it is therefore possible to solve the technical problems discussed at i) and ii) at the beginning of the description in a simple fashion which may be advantageously implemented without requiring a whole redesign of the machine 1, and which therefore is compatible with a retrofitting of already existing machines. Clearly, if the waste to be treated is dry waste, it is possible to deactivate the supply assembly 8 and to operate in a traditional fashion, which enables to avoid adapting other parameters of the work cycle if - as already stated in the foregoing - the moisture percentage in the waste is not high. Finally, the implementation of the hinge 4H described herein enables uniformizing the interference between the lid 22 and the associated gasket, thereby prolonging the useful life of the latter. In this regard, the technical effect of the hinges 4H may be extended also to machines that do not comprise the supply assembly 8; however, if such hinge(s) are applied to the machine 1 which includes the supply assembly 8, the effect is particularly beneficial since, if waste with a high moisture content is regularly treated, the degree of vacuum within the volume V2 - albeit mitigated by the supply assembly 8 - is generally higher in comparison with machines which systemically treat dry waste. In the latter case, the gasket is generally subjected to stresses; thus, the possibility of mitigating the wear thereof by means of the hinges 4H contributes to the general efficiency of the machine 1 in treating waste with a high moisture content.

Of course, the implementation details and the embodiments may amply vary with respect to what has been described and illustrated, without departing from the extent of the present invention, as defined by the annexed claims.

## Claims

1. A machine (1) for treating waste including:
- a treatment cell (2) configured to receive waste for treatment in a treatment volume (V2) thereof, the treatment cell (2) comprising a main axis (Z2), a loading opening (4) which is closable and arranged at a top end (2T) thereof, and a discharge opening (5) closable and arranged at a bottom end (2B) thereof, the bottom end (2B) and the top end (2T) being opposite ends along said main axis (Z2),
- a rotor (7) arranged in the treatment volume (V2) of said treatment cell (2) at the bottom end (2B) thereof, the rotor (Z2) being operable in rotation about said main axis (Z2) to heat and shred the waste in said treatment volume (V2),
- a heated air supply assembly (8) having an inlet port (IN) and a delivery port (OUT), the discharge port being (OUT) in fluid communication with the treatment volume (V2), the heated air supply assembly (8) comprising an air feeder device (9) configured to draw in an air flow rate from an environment outside of the treatment volume (V2) and a heater device (10, R) configured to increase the temperature of said air flow rate prior to entering said treatment volume (V2).

2. The machine (1) according to claim 1, wherein the delivery port (OUT) is in fluid communication with the treatment volume (V2) at said bottom end (2B).

3. The machine (1) according to claim 2, wherein the delivery port is in fluid communication with the treatment volume (V2) at a hub (H7) of said rotor (7).

4. The machine (1) according to claim 1, wherein said bottom end (2B) comprises:
- a lower disc (11) including at least one channel (12) extending towards said main axis (Z2) and formed within a thickness (T11) of said lower disc (11), wherein each channel (12) is in fluid communication with the outlet (OUT) of the heated air supply unit (8), is open at an upper face (13) of said lower disc (11), and leads out at a first through-hole (H11) of said lower disc (11) coaxial to the main axis (Z2),
- an upper disc (14) arranged above said lower disc (11) in such a way that a lower face (15) of the upper disc faces the upper face (13) of the lower disc (11) superiorly delimiting each channel (12), the upper disc (14) comprising a second through-hole (H14) coaxial to the main axis (Z2)
- a sleeve element (16) arranged coaxially to said main axis (Z2), the sleeve element (16) being housed in a sequence of holes comprising the first through-hole (H11) of the lower disc (11) and the second through-hole (H14) of the upper disc (14) and further comprising one or more distributor holes (16) in fluid communication with each channel (12) of the lower disc (11), each distributor hole (16) being configured for the introduction of the flow rate of air supplied by the heated air supply unit (8) into the treatment volume (V2),
wherein the sleeve element (16) is traversed by an output shaft (S7) of an electric motor (M7) connected in rotation to the hub (H7) of the rotor (7) for rotational drive of the rotor (7) itself.

5. The machine (1) according to any of the preceding claims, wherein said heater device (10, R) comprises at least one among:
- a heater (10), preferably an electric heater, arranged downstream of the air feeder device (9) along the fluid communication with the treatment volume (V2),
- a sheet-like resistor (R) arranged between the lower disc (11) and the upper disc (14).

6. The machine (1) according to any of the preceding claims, wherein the delivery port is in fluid communication with the treatment volume (V2) at a side wall (6) of said treatment cell between said bottom end (2B) and said top end (2T).

7. The machine (1) according to any one of the preceding claims, wherein said loading opening (4) is closable by means of a lid (22) hinged to a fixed support, particularly said treatment cell (2), by means of at least one lid hinge (4H), each lid hinge (4H) including:
- a first hinge element (25) coupled to said fixed support,
- a second hinge element (26) coupled to said cover (22),
- a hinge pin (27) received in said first hinge element (25) and in said second hinge element (26) and pivotally connecting them, the hinge pin (27) having a respective longitudinal axis (X22),
in which the hinge pin (27) is received in a floating manner in a direction transverse to the longitudinal axis (X22) thereof in at least one of said first hinge element (25) and second hinge element (26).

8. The machine (1) according to claim 7, wherein the first hinge member includes one or more rolling bearings (28) connected (29) to said fixed support, wherein the one or more rolling bearings (28) rotatably support the hinge pin (27) and are fixed in a transverse direction with respect to the longitudinal axis of the hinge pin (27).

9. The machine (1) according to claim 8, wherein the hinge pin (27) is received in said second hinge member (26) in a floating manner in a direction transverse to the longitudinal axis (X22) thereof, the hinge pin being further fixed in a rotation about the longitudinal axis of it (X22) relative to the second hinge member (26).

10. The machine (1) according to claim 9, wherein the hinge pin (27) comprises a pair of diametrically opposed flattenings (30, 31), wherein on a first flattening (30) a first screw (32) abuts that blocks a rotation of the hinge pin (27) relative to the hinge element (26) about the longitudinal axis (X22) of the hinge pin, and wherein on a second flattening (31) by a spring element (33) abuts that counters the movement of the hinge pin (27) in the direction transverse to the longitudinal axis (X22) thereof.
